# EUROPEAN PATENT APPLICATION

(11) **EP 3 834 798 A1**
(43) Date of publication of application: **16.06.2021**
(21) Application number: 20212661.1
(22) Date of filing: 09.12.2020
(51) Int. Cl.: A61H 3/00, A63B 22/02

(54) **WALKING ASSISTANCE SYSTEM AND CONTROL METHOD THEREFOR**

(30) Priority: 09.12.2019 JP 2019221878
(71) Applicant: Toyota Jidosha Kabushiki Kaisha, Aichi-ken, 471-8571 (JP)
(72) Inventor: MAEKITA, Tomoe, Aichi-ken, 471-8571 (JP); ODASHIMA, Tadashi, Aichi-ken, 471-8571 (JP); FUJINORI, Youichi, Aichi-ken, 471-8571 (JP); SUZUKI, Kanako, Aichi-ken, 471-8571 (JP); KOIKE, Uori, Aichi-ken, 471-8571 (JP)
(74) Representative: D Young & Co LLP

(57) **Abstract**

To provide a walking assistance system having a simple configuration with which a walking motion of a user can be appropriately assisted and a method therefor. The walking assistance system according to the present embodiments is a walking assistance system (2) worn on a leg part of a user and includes a damper (22) configured to apply a resisting force in a bending direction of a knee joint of the leg part, a sensor (4) configured to detect a switching timing in a gait cycle of the user, and a control unit configured to switch modes of the damper (22) in accordance with the switching timing so that a first mode and a second mode in which the resisting force is stronger than that in the first mode are repeated alternately.

## Description

### BACKGROUND

The present disclosure relates to a walking assistance system and a control method therefor.

Japanese Patent No. 5316708 discloses a walking support device for assisting walking motion. The walking support device includes an actuator and a one-way damper. The actuator applies a torque to a lower-leg link. The one-way damper generates a resisting force against the rotation in a knee flexing direction and does not generate a resisting force against the rotation in a knee extending direction. The one-way damper is controlled to repeat modes of a free mode in which the damper is allowed to rotate freely, a damper mode in which the damper applies a resisting force, and a lock mode in which the damper is prohibited from rotating in this order.

### SUMMARY

Since the walking assistance apparatus described above is fixed to a user's leg part, it is desired to reduce the size and the weight of the walking assistance apparatus. Further, it is desired that the walking assistance apparatus performs control so as to assist the walking motion even more appropriately.

A walking assistance system according to a first exemplary aspect is a walking assistance system worn on a leg part of a user, including: a damper configured to apply a resisting force in a bending direction of a knee joint of the leg part; a sensor configured to detect a switching timing in a gait cycle of the user; and a control unit configured to switch modes of the damper in accordance with the switching timing so that a first mode and a second mode in which the resisting force is stronger than that in the first mode are repeated alternately.

In the aforementioned walking assistance system, the damper may not be locked throughout the entire gait cycle.

In the aforementioned walking assistance system, the damper may be a one-way damper configured to move freely in an extending direction of the knee joint.

In the aforementioned walking assistance system, the sensor may be configured to measure a shin angle of the user.

In the aforementioned walking assistance system, the sensor may be configured to measure a distance from the sensor to a floor surface.

In the aforementioned walking assistance system, the damper may be configured to move freely so that the resisting force becomes 0 in the first mode.

It is desirable that the aforementioned walking assistance system does not include an actuator for generating an assisting force applied to the knee joint.

The aforementioned walking assistance system may further include a transmission unit configured to transmit data acquired by the walking assistance system to an external device.

The aforementioned walking assistance system is provided with a solar cell for supplying power to the sensor or the control unit, and a light receiving unit for the solar cell may be disposed on a surface of the walking assistance system.

A method for controlling a walking assistance system according to a second exemplary aspect is a method for controlling a walking assistance system worn on a leg part of a user that includes:
a damper configured to apply a resisting force in a bending direction of a knee joint of the leg part;
a sensor configured to detect a switching timing in a gait cycle of the user; and
a control unit configured to switch modes of the damper in accordance with the switching timing, the method including
controlling the walking assistance system so that a first mode and a second mode in which the resisting force is stronger than that in the first mode are repeated alternately.

In the aforementioned control method, the damper may not be locked throughout the entire gait cycle.

In the aforementioned control method, the damper may be a one-way damper configured to move freely in an extending direction of the knee joint.

In the aforementioned control method, the sensor may be configured to measure a shin angle of the user.

In the aforementioned control method, the sensor may be configured to measure a distance from the sensor to a floor surface.

In the aforementioned control method, the damper may be configured to move freely so that the resisting force becomes 0 in the first mode.

In the aforementioned control method, it is desirable that the walking assistance system does not include an actuator for generating an assisting force applied to the knee joint.

In the aforementioned control method, data acquired by the walking assistance system may be transmitted to an external device.

In the aforementioned control method, the walking assistance system may be provided with a solar cell for supplying power to the sensor or the control unit, and a light receiving part for the solar cell may be disposed on a surface of the walking assistance system.

According to the embodiments of the present disclosure, a walking assistance system having a simple configuration with which the walking motion can be appropriately assisted and a method therefor can be provided.

The above and other objects, features and advantages of the present disclosure will become more fully understood from the detailed description given hereinbelow and the accompanying drawings which are given by way of illustration only, and thus are not to be considered as limiting the present disclosure.

### BRIEF DESCRIPTION OF DRAWINGS

Fig. 1 is a perspective view schematically illustrating a configuration of a walking training system for which a walking assistance system according to an embodiment can be employed;
Fig. 2 is a front view schematically illustrating a configuration of the walking assistance system;
Fig. 3 is a side view schematically illustrating a configuration of the walking assistance system;
Fig. 4 is a block diagram showing a control system of the walking assistance system;
Fig. 5 is a diagram for describing a gait cycle and switching timings of the modes;
Fig. 6 is a block diagram showing a control system of the walking assistance system; and
Fig. 7 is a block diagram showing a control system of the walking assistance system according to a modified embodiment.

### DESCRIPTION OF EMBODIMENTS

Hereinafter, the present disclosure will be described through embodiments of the present disclosure. However, the embodiments are not intended to limit the scope of the present disclosure according to the claims. Further, not all of the components/structures described in the embodiments are necessarily indispensable as means for solving the problem. For clarifying the explanation, the following description and the drawings are partially omitted and simplified where appropriate. The same symbols are assigned to the same elements in the drawings and duplicated explanations thereof are omitted where appropriate.

### First Embodiment

A walking assistance apparatus (a walking assistance system) according to this embodiment is, for example, worn by a trainee who performs walking training. The trainee performs walking training with the walking assistance system worn on. For example, a trainee who is, for example, a hemiplegic patient suffering from paralysis in one of his/her legs wears the walking assistance system on his/her affected leg. In the following description, an example in which the trainee who is the user performs walking training in the walking training system at a late stage of rehabilitation will be described. Note that the walking assistance system is not limited for use in performing walking training in the walking training system and may be used, for example, when walking in a hospital or walking outdoors.

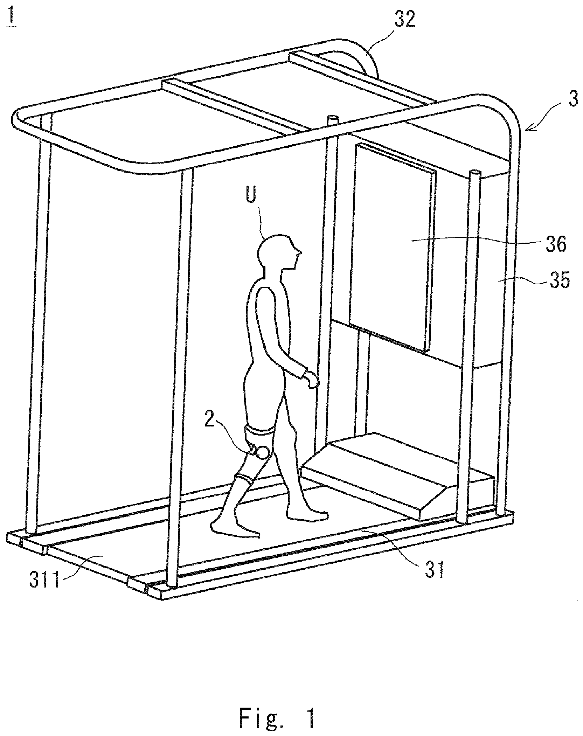

Fig. 1 is a schematic view for describing a configuration of a walking training system for which the walking assistance apparatus according to the first embodiment is employed. As shown in Fig. 1, a walking training system 1 includes a walking assistance apparatus (a walking assistance system) 2 worn on a leg part of a trainee U and a training apparatus 3 for performing walking training of the trainee U. Note that the walking assistance apparatus 2 may be used outside the training apparatus 3. That is, the walking assistance apparatus 2 may be used independently. For example, the trainee U who is wearing the walking assistance apparatus 2 may perform walking training on stairs, flatlands, slopes etc.

The walking assistance apparatus 2 is, for example, worn on the affected leg of the trainee U who performs walking training and assists the trainee U in walking. The trainee U performs walking training with the walking assistance apparatus 2 worn on his/her knee joint. The walking assistance apparatus 2 applies a resisting force in a flexing direction of the knee joint.

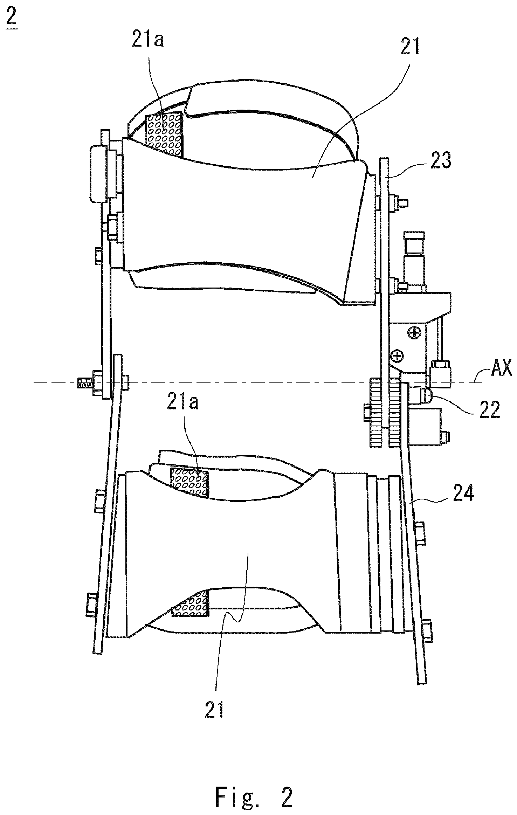

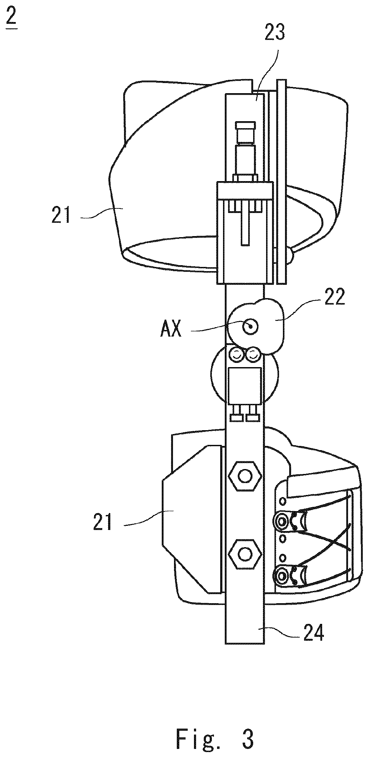

The walking assistance apparatus 2 will be described using Figs. 2 and 3. Fig. 2 is a front view of the walking assistance apparatus 2. Fig. 3 is a side view of the walking assistance apparatus 2.

The walking assistance apparatus 2 includes a supporter 21, a damper 22, an upper-leg frame 23, and a lower-leg frame 24. A short lower limb gear may be attached to the walking assistance apparatus 2 on a lower side thereof. The supporter 21 is formed of a stretchable material such as resin or fiber. The supporter 21 is worn on the knee joint and its surrounding, more specifically, from the upper leg across to the lower leg. Note that the upper leg refers to a part of the leg from the hip joint to the knee joint, and the lower leg refers to a part of the leg from the knee joint to the ankle joint. A part of the leg from the ankle joint to therebelow, that is, a part of the leg from the ankle joint to the toe side is referred to as the sole. The upper leg, the lower leg, and the sole are collectively referred to as the lower limb.

The supporter 21 includes a surface fastener 21a (hook-and-loop fastener such as Velcro tape) for wearing the walking assistance apparatus 2 on the knee joint. The trainee U wraps the supporter 21 around the leg part and fixes it with the surface faster 21a. The surface fastener 21a is provided to upper and lower parts of the knee joint, specifically, to a front side of the upper leg and a front side of the lower leg, respectively. The trainee U can easily wear/take off the walking assistance apparatus 2 using the surface fastener 21a. Further, it is possible to prevent the walking assistance apparatus 2 from slipping from the knee joint of the trainee U. The trainee U can adjust the degree of the feeling of tightness owing to the surface fastener 21a. Further, a fixing band may be provided in order to prevent the surface fastener 21a from being unfastened or the supporter 21 from slipping.

The upper-leg frame 23 and the lower-leg frame 24 are attached to a side part of the supporter 21. The upper-leg frame 23 is arranged along the upper leg. The lower-leg frame 24 is arranged along the lower leg. The upper-leg frame 23 and the lower-leg frame 24 are connected with each other through the damper 22. The damper 22 is, for example, a rotary damper, and is arranged at a side part of the knee joint. Specifically, the damper 22 is disposed at a height of the knee joint so that a rotation axis Ax of the damper 22 roughly coincides with the axis of the knee joint. The upper-leg frame 23 and the lower-leg frame 24 configure a link mechanism that is rotatable about the rotation axis Ax of the damper 22.

The damper 22 applies the resisting force in the flexing direction of the knee joint. For example, the damper 22 reduces its speed of rotation in the flexing direction of the knee joint by utilizing the viscous drag of fluid such as oil. It is desirable that the damper 22 is a one-way damper that applies the resisting force in only one direction. The damper 22 is configured to move freely so as not to apply the resisting force in the extending direction of the knee joint. As described later, the damper 22 can be switched using a switch.

Note that fixing of each of the upper-leg frame 23 and the lower-leg frame 24 is not limited to the use of the surface fastener. For example, the upper-leg frame 23 and the lower-leg frame 24 may be fixed to the upper leg and the lower leg, respectively, using a fixing means such as a belt, a button, a pin, or a band. The trainee U can still wear the walking assistance apparatus 2 even when the fixing means described above is employed.

Note that the aforementioned configuration of the walking assistance apparatus 2 is a mere example and it is not to be limited thereto. The walking assistance apparatus 2 may have any configuration as long as it is worn on the leg part of the trainee U and includes the damper 22.

Reference is made once again to Fig. 1. The training apparatus 3 includes a treadmill 31, a frame main body 32, a control device 35, and a display unit 36. The treadmill 31, the control device 35, and the display unit 36 are fixed to the frame main body 32. The display unit 36 is disposed in front of the trainee U.

The treadmill 31 includes a rotatable ring-like belt conveyor 311 where the trainee U walks, and rotates the belt conveyor 311 at a set speed Vs. The trainee U stands on the belt conveyor 311 and walks thereon in accordance with the movement of the belt conveyor 311. The display unit 36 displays information such as a training instruction to the trainee U, a training menu, and training information (the set speed, the biological information etc.). For example, the display unit 36 may include a touch panel, in which case the trainee U can input various information through the display unit 36. Further, the training apparatus 3 may include a camera or the like for picking up an image of the trainee U. By this configuration, the display unit 36 can display an image of the walking motion of the trainee U who is under training.

The control device 35 has a hardware configuration having a microcomputer at the center thereof, the microcomputer being configured of, for example, a CPU (Central Processing Unit) that performs operation processing, control processing etc., a ROM (Read Only Memory) that stores an operation program, a control program, and the like executed by the CPU, a RAM (Random Access Memory) that stores various data, and an interface (I/F) for inputting/outputting a signal from/to the outside. The CPU, the ROM, the RAM, and the interface unit are connected with one another through, for example, a data bus.

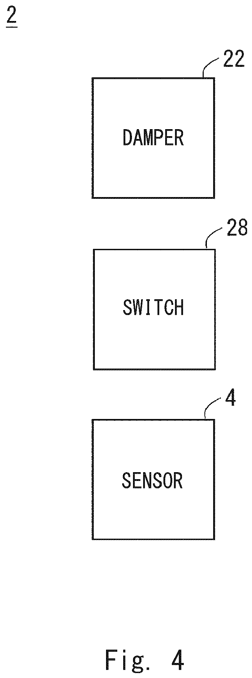

Fig. 4 is a block diagram showing a control system of the walking assistance apparatus 2 according to this embodiment. The walking assistance apparatus 2 includes the damper 22, a sensor 4, and a switch 28.

The damper 22 applies the resisting force in the flexing direction of the knee joint as described above. The switch 28 switches the modes of the damper 22. For example, a solenoid switch can be used as the switch 28. The switch 28 switches the modes of the damper 22 so that the first mode and the second mode are repeated alternately. In the first mode, the damper 22 is turned off to be brought into the free mode in which the resisting force is not applied in the knee flexing direction. In the second mode, the damper 22 is turned on to be brought into the damper mode in which the resisting force is applied in the knee flexing direction.

The sensor 4 detects a timing in the walking motion of the trainee U. Specifically, the sensor 4 is provided for detecting a switching timing in a gait cycle (a gait frequency). The switch 28 switches the modes of the damper 22 based on the result of detection by the sensor 4. That is, the ON/OFF control of the solenoid switch as the switch 28 is performed based on a timing signal output from the sensor 4. By this configuration, the modes of the damper 22 are switched by the switch 28 at a constant timing in the gait cycle. Power is supplied to the sensor 4 and the switch 28 from a battery (not shown) loaded on the walking assistance apparatus 2.

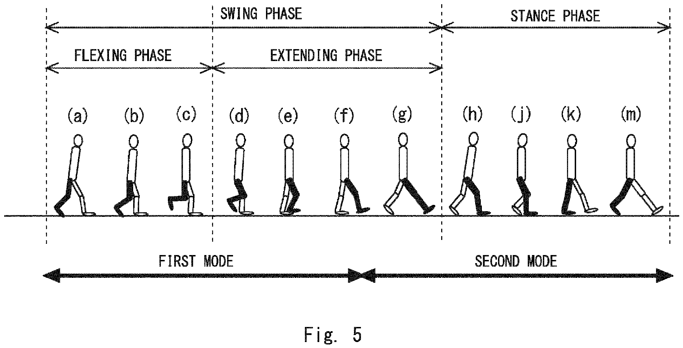

Fig. 5 is a diagram showing a walking motion in one gait cycle and timings at which the modes are switched. Note that one gait cycle includes two steps, that is, one left-leg step and one right-leg step. In Fig. 5, one gait cycle is shown in the order of timings (a) to (m). After the timing (m), the timing returns to the timing (a) to start the next gait cycle. In Fig. 5, the timings from (a) to (g) are in a swing phase, and the timings from (h) to (m) are in a stance phase. Between the timing (g) and the timing (h), the sole comes in contact with the ground, and between the timing (m) back to the timing (a), the sole comes off the ground. The timings (a) to (c) are in a flexing phase during which a flexing angle of the knee joint increases, and the timings (d) to (g) are in an extending phase during which the flexing angle of the knee joint decreases. Note that the affected leg on which the walking assistance apparatus 2 is worn is used as a reference as regards the swing phase, the stance phase, the flexing phase, and the extending phase.

In this embodiment, the mode is switched from the first mode to the second mode in the extending phase, specifically, at the timing (f). Further, the mode is switched from the second mode to the first mode at the timing when the phase shifts from the stance phase to the swing phase, specifically, between the timing (m) and the timing (a). In the swing phase, there is no need to support the weight of the trainee U with the affected leg. Therefore, there is no need to generate the resisting force against the knee joint with the damper, and thus the damper can move freely with respect to the flexing direction throughout most of the swing phase. In other words, the mode throughout the entire stance phase is the second mode in which the damper 22 generates the resisting force in the flexing direction.

Further, only the first mode in which the damper moves freely and the second mode in which the damper generates the resisting force are set in one gait cycle, and the switch 28 switches the modes alternately between the first mode and the second mode during the walking motion. That is, the switch 28 controls ON/OFF of the damper 22 based on the timing signal. Thus it is possible to perform appropriate control with a simple configuration. For example, when the walking motion varies between each gait cycle, the switching timings detected by the sensor 4 may vary. Even in this case, at the timing before switching from the swing phase to the stance phase, that is, at an arbitrary timing in the extending phase, the damper 22 need only be switched from the first mode to the second mode.

Specifically, between the timings (d) to (g), the switch 28 need only to switch the modes. Since the margin for the errors in the switching timings detected by the sensor can be widened, an appropriate control can be performed. Further, in the extending phase, the knee joint gradually rotates in the extending direction from the flexed state. Therefore, even when the damper 22 is in the second mode, no resisting force is generated. Thus it is possible to switch the modes without causing the damper 22 to interrupt the walking motion of the trainee U.

Further, in the extending direction, the damper moves freely throughout the entire gait cycle, and thus the knee joint of the trainee U can be extended freely. Furthermore, since there is no lock mode for locking the damper 22 between the damper mode and the free mode, it is possible to prevent the damper 22 from interrupting the walking motion. Since the mode shifts from the second mode to the first mode without shifting to the lock mode, it is possible to easily perform appropriate control.

Note that in the aforementioned description, the modes are switched between the first mode and the second mode by performing ON/OFF control of the damper 22 using the switch 28, although the control of switching the modes is not limited to performing the ON/OFF control of the damper 22. The resisting force of the damper 22 may be made to gradually increase or decrease at the timings at which the modes are switched. For example, a period during which the resisting force gradually changes may be set between the first mode and the second mode. For example, when switching between the first mode and the second mode, the resisting force may be made to gradually increase or decrease.

Further, the first mode is not limited to a free mode of the damper 22. For example, the resisting force of the damper 22 in the first mode should only be smaller than the resisting force of the damper 22 in the second mode. Therefore, the damper 22 may or may not generate the resisting force in the first mode.

The switching timings between the swing phase and the stance phase may be detected in accordance with the output value of the sensor 4, the first mode being set for the swing phase and the second mode being set for the stance phase. That is, the timing at which the phase shifts from swing phase to the stance phase and the timing at which the phase shifts from the stance phase to the swing phase may be the switching timings of the modes of the damper 22. The switch 28 is a control unit that alternately switches the modes between the first mode and the second mode.

Further, the walking assistance apparatus 2 does not require an actuator for generating the resisting force against the knee joint. This means that there is no need to provide a motor and an actuator for applying the assisting force, and thus the walking assistance apparatus 2 can be reduced in size and weight. Further, by using an oil damper having a passive configuration, it becomes easier to follow the walking motion of the trainee U, and thus the control of the walking assistance apparatus is facilitated.

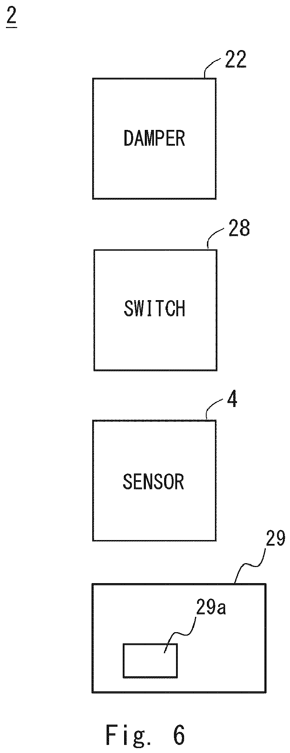

Further, the walking assistance apparatus 2 may be equipped with a battery 29 for supplying power to the sensor 4 and the switch 28 as shown in Fig. 6. Since a solenoid switch having a low power consumption is mounted on the walking assistance apparatus 2 as the switch 28, even when a low-capacitance battery is used as the battery 29, it is possible to supply power to the switch 28 and the sensor 4. Accordingly, the weight and the size of the battery 29 can be reduced. The battery 29 may be a secondary battery that can be charged/discharged, or may be a primary battery. Further, the battery 29 may be a solar cell. In this case, a light receiving part 29a for the solar cell need only be provided on a surface of the walking assistance apparatus 2. Further, the switch 28 may include a switch other than the solenoid switch. For example, the switch 28 may include a switch such as a semiconductor switch. The switch 28 may be configured such that the modes of the damper 22 are switched by controlling the ON/OFF switching of the switch in accordance with the control signal from an electronic circuit. Further, by employing the solenoid switch for the control unit for switching the modes, it is possible to reduce the size and the weight of the walking assistance apparatus.

### (Specific example of sensor 4)

Various types of sensors can be used as the sensor 4. Specific examples of the sensor 4 will be described below.

An angle sensor for detecting a shin angle (a lower limb angle) and an angular velocity sensor for detecting an angular velocity of the shin (the lower limb) can be used as the sensor 4. The angle sensor and the angular velocity sensor which are attached to the lower-leg frame 24 can be used as the sensor 4. The sensor 4 measures the shin angle since the shin angle changes in accordance with the timing in the gait cycle. The detection result of the shin angle indicates a waveform corresponding to the gait cycle. That is, the shin angle varies periodically in accordance with the gait cycle. Based on the shin angle measured by the sensor 4, the walking timing can be detected.

For example, the output value of the sensor 4 is compared with the threshold value, and the modes of the damper 22 may be switched in accordance with the result of the comparison. For example, the modes are switched in accordance with a timing signal indicating the timing at which the output value of the sensor 4 exceeds the threshold value or a timing signal indicating the timing at which the threshold value falls below the threshold value. Note that the first threshold value for detecting the switching timing from the first mode to the second mode and the second threshold value for detecting the switching timing from the second mode to the first mode may be set.

Alternatively, a distance measurement sensor for measuring a distance from the floor surface (for example, the belt conveyor 311 of the treadmill) to the sensor 4 can be used. For example, a distance measurement sensor attached to a shoe or a sole or in the vicinity thereof can be used as the sensor 4. Since the distance from the sole to the floor surface varies in accordance with the walking motion, the waveform corresponding to the gait cycle is indicated. That is, the distance from the sole to the floor surface varies periodically in accordance with the gait cycle. Therefore, the walking timing can be detected by using the sensor 4 as the distance measurement sensor. For example, the modes of the damper 22 may be switched in accordance with the result of comparison between the output value of the sensor 4 and the threshold value. An optical sensor can be used as the distance measurement sensor.

Further, the switching timings may be detected using a plurality of sensors 4 in combination. For example, the sensor 4 may include both of a first sensor for detecting the shin angle and a second sensor for detecting the distance from the sole to the floor surface. The specific examples of the sensor 4 are not to be limited to the aforementioned examples. It is desirable that the sensor 4 is mounted on the walking assistance apparatus 2. Alternatively, the sensor 4 may be mounted outside the walking assistance apparatus 2. For example, the sensor 4 may be a camera, a depth sensor, a distance measurement sensor etc. attached to the training apparatus 3. When the sensor 4 is disposed outside the walking assistance apparatus 2, the walking assistance apparatus 2 need only include a reception unit that receives a signal indicating the switching timing from the externally-disposed sensor 4.

### Modified Example

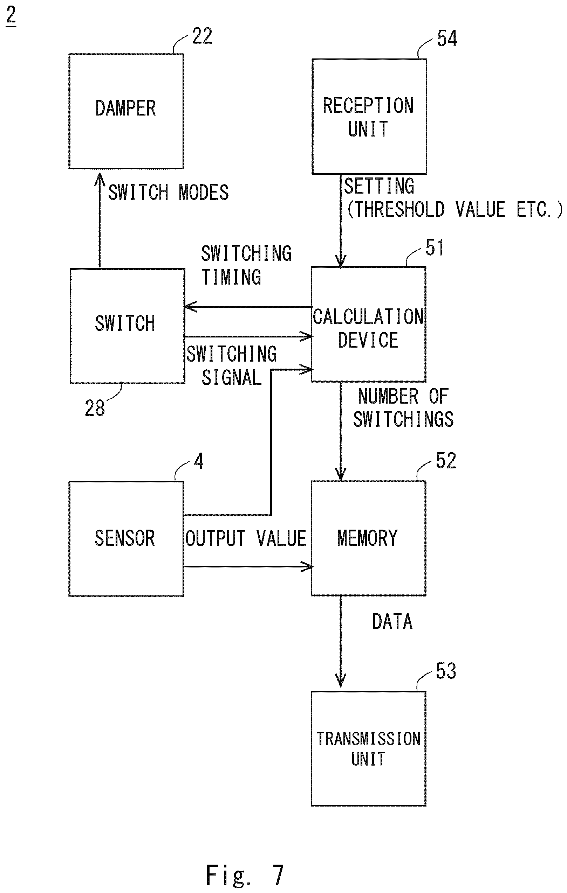

Hereinbelow, a modified example of the walking assistance apparatus according to the present embodiment will be described by referring to Fig. 7. Fig. 7 is a block diagram showing a control system of the walking assistance apparatus 2 according to a modified example. In Fig. 7, in addition to the configuration shown in Fig. 4, the walking assistance apparatus 2 includes a calculation device 51, a memory 52, a transmission unit 53, and a reception unit 54. Note that the damper 22, the switch 28, and the sensor 4 are the same as those of the first embodiment, and thus explanations thereof are omitted.

The calculation device 51 has, for example, a hardware configuration having a microcomputer at the center thereof, the microcomputer being configured of a CPU (Central Processing Unit) that performs operation processing, control processing etc., a ROM (Read Only Memory) that stores an operation program, a control program and the like executed by the CPU, a RAM (Random Access Memory) that stores various data, and an interface (I/F) that inputs/outputs a signal to/from the outside. The CPU, the ROM, the RAM, and the interface unit are connected with one another through, for example, a data bus.

The output value of the sensor 4 is input to the calculation device 51. The calculation device 51 detects the switching timing in the gait cycle by performing prescribed operation processing to the output value of the sensor 4. For example, the calculation device 51 detects the switching timing by comparing the output value of the sensor with the threshold value. When the calculation device 51 outputs the switching timing to the switch 28, the switch 28 switches the modes of the damper 22. The memory 52 stores therein the data of the output value of the sensor 4 which is output during the walking training.

A switching signal indicating that the modes have been switched may be input from the switch 28 to the calculation device 51. The calculation device 51 counts how many times the switching operations are performed by the switch 28 based on the switching signal. The calculation device 51 counts how many times the switching operations are performed by the switch 28 and writes the number of switching operations into the memory 52. The number of switching operations performed by the switch 28 corresponds to the number of steps taken by the trainee. By this configuration, the trainee can count the number of steps he/she has taken during the walking training without having to wear a pedometer (registered trademark) or the like. Accordingly, the convenience of the walking assistance apparatus can be improved. Further, the trainee can count the number of steps he/she has taken during the walking training without having to wear an additional sensor for counting the number of steps. Therefore, the cost and the size of the walking assistance apparatus 2 can be reduced in comparison to the configuration in which the sensor for counting the number of steps is additionally provided.

The transmission unit 53 transmits data to external devices such as the control device 35 and an external server. The reception unit 54 receives data from the external devices. The transmission unit 53 and the reception unit 54 transmit/receive data in accordance with a communication standard such as Bluetooth (registered trademark). Both the transmission unit 53 and the reception unit 54 may perform communication by radio communication or wired communication.

The transmission unit 53 transmits data acquired by the walking assistance apparatus 2, such as the output value of the sensor 4 during the walking training and the number of switching operations performed by the switch 28, to the external devices. By this configuration, the external devices can collect data. Accordingly, it becomes possible to cooperate with the external devices and to reduce the capacity of the memory 52. Further, the transmission unit 53 may automatically transmit the output of the sensor 4 during the training.

The reception unit 54 receives data from the external device such as the control device 35. By this configuration, the conditions for detecting the walking timing can be changed. Specifically, the setting of the threshold value for detecting the switching timing etc. can be changed. For example, when changing the setting of the threshold value, the control device 35 may transmit an instruction to change the setting of the threshold value. When the reception unit 54 receives the instruction to change the setting of the threshold value, the calculation unit 51 changes the threshold value. By this configuration, the most suitable detection conditions can be set for each user. Thus the most suitable detection conditions can be set for each user.

For example, the transmission unit 53 transmits the output value of the sensor to the control device 35. The display unit 36 or the like displays a periodical waveform of the sensor output. A training assistant such as a physical therapist operates an input device such as a touch panel, a keyboard, a switch, or a mouse while checking the waveform of the sensor output, whereby the threshold value can be set.

Note that the present disclosure is not limited to the above embodiments and may be changed as appropriate without departing from the spirit of the present disclosure.

The program can be stored and provided to a computer using any type of non-transitory computer readable media. Non-transitory computer readable media include any type of tangible storage media. Examples of non-transitory computer readable media include magnetic storage media (such as floppy disks, magnetic tapes, hard disk drives, etc.), optical magnetic storage media (e.g. magneto-optical disks), CD-ROM (compact disc read only memory), CD-R (compact disc recordable), CD-R/W (compact disc rewritable), and semiconductor memories (such as mask ROM, PROM (programmable ROM), EPROM (erasable PROM), flash ROM, RAM (random access memory), etc.). The program may be provided to a computer using any type of transitory computer readable media. Examples of transitory computer readable media include electric signals, optical signals, and electromagnetic waves. Transitory computer readable media can provide the program to a computer via a wired communication line (e.g. electric wires, and optical fibers) or a wireless communication line.

From the disclosure thus described, it will be obvious that the embodiments of the disclosure may be varied in many ways. Such variations are not to be regarded as a departure from the spirit and scope of the disclosure, and all such modifications as would be obvious to one skilled in the art are intended for inclusion within the scope of the following claims.

## Claims

1. A walking assistance system (2) worn on a leg part of a user, comprising:
a damper (22) configured to apply a resisting force in a bending direction of a knee joint of the leg part;
a sensor (4) configured to detect a switching timing in a gait cycle of the user; and
a control unit (28) configured to switch modes of the damper (22) in accordance with the switching timing so that a first mode and a second mode in which the resisting force is stronger than that in the first mode are repeated alternately.

2. The walking assistance system (2) according to Claim 1, wherein the damper (22) is not locked throughout the entire gait cycle.

3. The walking assistance system (2) according to Claim 1 or 2, wherein the damper (22) is a one-way damper configured to move freely in an extending direction of the knee joint.

4. The walking assistance system (2) according to any one of Claims 1 to 3, wherein the sensor (4) is configured to measure a shin angle of the user.

5. The walking assistance system according to any one Claims 1 to 4, wherein the sensor (4) is configured to measure a distance from the sensor (4) to a floor surface.

6. The walking assistance system according to any one of Claims 1 to 5, wherein the damper (22) is configured to move freely so that the resisting force becomes 0 in the first mode.

7. The walking assistance system (2) according to any one of Claims 1 to 6, wherein the walking assistance system (2) does not include an actuator for generating an assisting force applied to the knee joint.

8. The walking assistance system according to any one of Claims 1 to 7, further comprising a transmission unit (53) configured to transmit data acquired by the walking assistance system (2) to an external device.

9. The walking assistance system according to any one of Claims 1 to 8, wherein
the walking assistance system is provided with a solar cell for supplying power to the sensor or the control unit (28), and
a light receiving part for the solar cell is disposed on a surface of the walking assistance system.

10. A method for controlling a walking assistance system worn on a leg part of a user that includes:
a damper (22) configured to apply a resisting force in a bending direction of a knee joint of the leg part;
a sensor (4) configured to detect a switching timing in a gait cycle of the user; and
a control unit (28) configured to switch modes of the damper in accordance with the switching timing, the method comprising
controlling the walking assistance system so that a first mode and a second mode in which the resisting force is stronger than that in the first mode are repeated alternately.

11. The method for controlling the walking assistance system according to Claim 10, wherein the damper (22) is not locked throughout the entire gait cycle.

12. The method for controlling the walking assistance system according to Claim 10 or 11, wherein the damper (22) is a one-way damper configured to move freely in an extending direction of the knee joint.

13. The method for controlling the walking assistance system according to any one of Claims 10 to 12, wherein the sensor (22) is configured to measure a shin angle of the user.

14. The method for controlling the walking assistance system according to any one of Claims 10 to 13, wherein the sensor (4) is configured to measure a distance from the sensor (4) to a floor surface.

15. A computer to execute the method according to any one of Claims 10 to 14.
